# EUROPEAN PATENT SPECIFICATION

(11) **EP 2 790 760 B1**
(45) Date of publication and mention of the grant of the patent: **09.03.2016**
(21) Application number: 12808337.5
(22) Date of filing: 13.12.2012
(51) Int. Cl.: A61M 15/00

(54) **A DEVICE FOR ADMINISTERING A POWDERED MEDICAMENT TO A PATIENT BY INHALATION**
VORRICHTUNG ZUR ABGABE EINES PULVERFÖRMIGEN MEDIKAMENTS AN EINEN PATIENTEN MITTELS INHALATION
DISPOSITIF D'ADMINISTRATION D'UN MÉDICAMENT EN POUDRE À UN PATIENT PAR INHALATION

(30) Priority: 16.12.2011 EP 11194087
(43) Date of publication of application: 22.10.2014
(73) Proprietor: Sanofi SA, 1214 Vernier (CH)
(72) Inventor: MAYER, Stefan, 79098 Freiburg im Breisgau (DE)
(74) Representative: Epping - Hermann - Fischer
(86) International application number: PCT/EP2012/075428
(87) International publication number: WO 2013/087788

(56) References cited:
- EP-A1- 2 277 576
- WO-A1-2007/132217
- WO-A1-2008/008021
- DE-A1-102009 041 664
- US-A1- 2004 025 877
- US-A1- 2010 018 527
- US-A1- 2010 309 020

## Description

The present description pertains to a device for administering a powdered medicament to a patient by inhalation. These devices comprise so-called dry powder inhalers (DPIs). Such devices are for example shown in documents US 2010/309020 A1 or WO 2007/132217 A1.

Known devices require sophisticated mechanisms for provisioning the powdered medicament such that the powdered medicament can be administered to the patient. Additionally, the devices must comply with safety standards. Therefore, safety mechanisms must be implemented in a device to secure the device against, and to protect a user from, unintended administration and/or erroneous dosage of the powdered medicament. Moreover, known devices usually require a large amount of parts to provide the desired functionality.

It is an object of the present disclosure to describe a device of the kind mentioned above which is cost-effective, easy to use and provides for a reliable and comfortable administration of a powdered medicament to a patient.

The device comprises a body, an internal housing in the body, an inhalation channel, a mouthpiece, the inhalation channel being provided to communicate with the mouthpiece, and a dose provisioning member. The dose provisioning member comprises at least one cavity for accommodation of a powdered medicament, the dose provisioning member further being enabled to adopt a first position in which the cavity is out of alignment with the inhalation channel and a second position in which the cavity is in alignment with the inhalation channel.

According to a first embodiment, the device as introduced above is characterized in that in the first position of the dose provisioning member a part of the dose provisioning member lies in the inhalation channel such that the inhalation channel is at least partly covered by a wall of the dose provisioning member. The dose provisioning member may be arranged such that the cross-section of the inhalation channel is partly or fully covered.

The dose provisioning member may fulfill at least two functionalities. A first functionality is given by the fact that the dose provisioning member only allows an administration of the powdered medicament to the patient when the dose provisioning member is in its second position, i.e. when the cavity is in alignment with the inhalation channel. In the first position of the dose provisioning member, provisioning of the powdered medicament to the patient and a subsequent inhalation of the powdered medicament by the patient is prevented, as the cavity is out of alignment with the inhalation channel.

Moreover, the second functionality of the dose provisioning member is given by the fact that the dose provisioning member covers, partly or fully, the cross-section of the inhalation channel. Hence, the dose provisioning member may block the inhalation channel, when the dose provisioning member is in its first position. This means, in the first position of the dose provisioning member an inhalation by a patient using the device cannot be executed since no air, not to mention any powdered medicament, can flow through the inhalation channel towards the mouthpiece of the device.

Thus, the device has a twofold safety mechanism which may be implemented by just one single piece, i.e. the dose provisioning member. In the first position of the dose provisioning member both the provision of the powdered medicament to the patient and an inhalation through the inhalation channel in the direction of the mouthpiece is prevented. An unintentional administration of a powdered medicament as well as an activation of the device due to an inhalation by the patient without the powdered medicament being prepared for inhalation is prevented.

The design of the dose provisioning member as explained above allows a reduction of the number of parts within the device and therefore a reduction of the overall costs of the device.

According to a second embodiment, which may be an alternative embodiment to the first embodiment or which may provide features in addition to the first embodiment, the device is characterized in that it comprises at least one cavity, i.e. one or more cavities, for accommodation of a powdered medicament, wherein each cavity is prefilled with a single dose of a powdered medicament and wherein the powdered medicament is solely stored in each cavity and wherein the device is free of a powder reservoir for storing a plurality of doses.

The term "prefilled" means that each cavity is already filled with powder before the first use of the device. Each cavity contains a single dose of a powdered medicament. This means, the device provides one or more cavities filled with a predetermined amount, dose or "shot" of the powdered medicament, wherein each cavity can be brought in alignment with the inhalation channel by movement of the dose provisioning member with respect to the inhalation channel such that the powdered medicament contained in the cavity can be brought into a position, where it is ready for inhalation by a patient. In this way a certain number of single doses may already be prepared when a patient needs to use the device and needs to inhale the powdered medicament.

A powder reservoir for storing a plurality of doses in the device is not required. This means, the device can be designed as a compact and small device without additional space for storing the powdered medicament. Moreover, an additional mechanism for gathering a predetermined amount, i.e. a dose, of a powdered medicament and for separating the predetermined amount from the powder within a powder reservoir, is not necessary.

A device according to the second embodiment can also be designed with a small number of parts, wherein single doses of a powdered medicament are prepared for being provided to a patient. Such a device is cost-effective and easy to use. By movement of the dose provisioning member from its first position to its second position as explained above, a cavity prefilled with a single dose of a powdered medicament can be brought in alignment with the inhalation channel such that the inhalation and the subsequent administration of the powdered medicament to the patient can be executed in an easy manner.

In the following, embodiments and features relating to the first and the second embodiment of the device, as explained above, are presented.

In an embodiment, the device is further characterized in that in the first position of the dose provisioning member the cavity is covered by inner walls of the housing such that the powdered medicament is enclosed by the cavity and the inner walls of the housing. This means, that in the first position of the dose provisioning member a powdered medicament which is prefilled in the cavity can be prevented from falling out of the cavity.

In this context, the term "cavity" may mean any recess or hole in the dose provisioning member that enables the accommodation of the powdered medicament. This means that the cavity is formed by walls within the dose provisioning member, thereby providing at least one opening such that the powdered medicament can leave the cavity.

In the first position the cavity may be covered by inner walls of the housing within the device such that the opening of the cavity is closed by these inner walls of the housing. This means, that the inner walls of the housing together with the walls of the cavity within the dose provisioning member may provide a closed chamber in which the powdered medicament is contained and enclosed. In this way, the powdered medicament can be protected from environmental influences. Moreover, the powdered medicament is secured against leaving the cavity, such that an unintentional distribution of the powdered medicament within the device is prevented.

In an embodiment, the cavity extends through the dose provisioning member, wherein in the second position of the dose provisioning member the cavity lies in the inhalation channel such that a cross-section of the cavity taken along the direction of movement of the dose provisioning member between the first and the second position is in alignment with the cross-section of the inhalation channel. The design of the cavity may provide a hole extending through the walls of the dose provisioning member. In this way the cavity provides a kind of channel or tunnel through the dose provisioning member. This means the dose provisioning member has at least two openings to the outside such that air may flow, not only into, but also through the cavity for entraining the powdered medicament out of the cavity.

The cavity can be positioned in the inhalation channel such that a cross-section of the cavity taken along the direction of movement of the dose provisioning member between the first and the second position is in alignment with the cross-section of the inhalation channel. In this way, the powdered medicament can enter the inhalation channel. Moreover, air may flow through the inhalation channel and through the cavity of the dose provisioning member, when the cavity lies in the inhalation channel. Hence, an air-powder-mixture can be created by air flowing through the inhalation channel and entraining the powder out of the cavity.

The design of the cavity as explained above provides for a good and effective depletion or emptying of the cavity, such that all of the powdered medicament can be administered to the patient during one inhalation cycle. This may help to better administer a certain dose of a powdered medicament to the patient and may provide an exact dosage of the powdered medicament.

In the second position of the dose provisioning member with the cavity lying in the inhalation channel as explained above, the cavity may be enclosed partly by movable walls which may be movable relative to the dose provisioning member such that the powdered medicament is protected from entering the inhalation channel until an inhalation of the medicament is triggered by a patient. In this case the movable walls may slide in a release position thereby releasing the cavity such that the powdered medicament can flow into the inhalation channel.

According to an embodiment a protective member is provided within the housing, the protective member being enabled to adopt a first position in which a part of the protective member lies in the inhalation channel such that the cross-section of the inhalation channel is covered by a wall of the protective member and to adopt a second position in which the protective member does not lie in the inhalation channel such that the cross-section of the inhalation channel is not covered by a wall of the protective member.

The protective member is further simple but effective safety mechanism which prevents an unintentional inhalation or actuation of the device. When the protective member lies in the inhalation channel, the inhalation channel is blocked such that no inhalation can be effected by a patient. This mechanism is similar to the first embodiment as explained above wherein in the first position of the dose provisioning member a part of the dose provisioning member lies in the inhalation channel such that the cross-section of the inhalation channel is covered by a wall of the dose provisioning member. Both the dose provisioning member and the protective member seen individually provide a safety mechanism such that the device can only be actuated if intended by a user.

The device can be designed such that in the first position of the protective member, the protective member encloses a part of the dose provisioning member when the dose provisioning member is in its first position, whereby the protective member encloses the cavity of the dose provisioning member when the dose provisioning member is in its second position. This design illustrates the double safety mechanism of the device. When the dose provisioning member is in its first position, the inhalation channel can be covered by a wall of the dose provisioning member such that no air may flow through the inhalation channel. Additionally, the protective member encloses the part of the dose provisioning member within the inhalation channel such that the inhalation channel is additionally blocked and covered by the protective member.

In the case that the dose provisioning member is moved from the first position to the second position, such that the cavity is in alignment with the inhalation channel, the protective member is still in its first position such that the cavity filled with the powdered medicament and being positioned in the inhalation channel is covered by the protective member. This means, the powdered medicament within the cavity remains enclosed by the walls of the cavity of the dose provisioning member as well as by walls of the protective member such that in the second position of the dose provisioning member with the protective member being in its first position, a closed chamber remains formed in the inhalation channel enclosing the powdered medicament. Hence, the protective member may act as movable walls of the kind as explained above.

The chamber which is formed by the cavity and the protective member remains closed until the protective member is moved from its first position into its second position in which the protective member does not lie in the inhalation channel. In the latter case the cavity of the dose provisioning member lies in the inhalation channel such that powdered medicament may enter from the cavity into the inhalation channel.

Consequently, the device can only be actuated if firstly the dose provisioning member is moved from its first initial position to its second position, thereby bringing the cavity filled with powdered medicament in alignment with the inhalation channel and if, subsequently, the protective member is moved from its first initial position to its second position, thereby freeing the inhalation channel and exposing the cavity such that powdered medicament can enter the inhalation channel. In this way, the device is made even more secure and reliable.

In an embodiment, the protective member is designed to be moved from the first position into the second position due to a predetermined pressure difference applicable by a user via the mouthpiece. This means that the protective member is actuated only when a patient sucks on the device and provides an under-pressure by inhalation such that a pressure difference is created. This pressure difference triggers the protective member to be moved from its first position to its second position.

In an embodiment, the protective member is guided by a guide member wherein a predetermined friction is provided at a contact area between the protective member and the guide member such that a movement of the protective member from the first position into the second position is only allowed if the pressure difference exceeds a predetermined threshold. This means that the protective member is not moved from its first position to its second position when a user only insufficiently sucks on the device. This effect can prevent an unintentional inhalation, since an inhalation is prevented when a user is breathing in with small inhalation action but still does not want to strongly inhale the dose of the powdered medicament or is still not ready for such an inhalation.

Moreover, the mechanism allows a good and reliable transportation of the powdered medicament to the target place, e.g. the lungs of the patient, and may help to prevent that the powdered medicament is insufficiently transported, e.g. only into the mouth region or the oral mucosa instead of the lungs due to an insufficient inhalation action. Furthermore, a strong inhalation action provides a better opening of the alveoli within the lungs of the patient. This means, the stronger the inhalation action by the patient, the better the absorption of the powdered medicament by the alveoli within the lungs of the patient.

By triggering the actuation of the protective member due to a predetermined pressure threshold, the absorption level of the powdered medicament within the alveoli of the lungs can be controlled. Consequently, the design of the protective member and the guide member with a predetermined friction between the two members effects a control of the absorption of the powdered medicament in the lungs of the patient. This results in a better and more effective administration of the powdered medicament to the patient.

In an embodiment, the dose provisioning member and the protective member interact with each other, such that in the first position of the dose provisioning member the actuation force required for displacing the protective member from its first position to its second position is greater than in the second position of the dose provisioning member.

The interaction of the dose provisioning member and the protective member in the first position of the dose provisioning member provides another safety mechanism, wherein the actuation of the protective member is only allowed if the dose provisioning member is placed in its second position with the cavity being placed within the inhalation channel. This means that the protective member cannot be actuated when no powder is placed within the inhalation channel for provisioning the powder to the patient. Moreover, this safety mechanism prevents an unintentional actuation of the protective member, e.g. during transportation of the device.

According to one embodiment the interaction between the protective member and the dose provisioning member can be obtained by hook-shaped members interacting with each other. According to another embodiment, the interaction between the protective member and the dose provisioning member can be obtained by a widened cross-section of the dose provisioning member at the part of the dose provisioning member lying in the inhalation channel when the dose provisioning member is in its first position such that this part of the dose provisioning member is in contact with the protective member. In this way the widened cross-section of the dose provisioning member can effect a pressure force against the walls of the protective member within the inhalation channel such that a movement of the protective member against this pressure force is inhibited. "Inhibited" means that a movement is possible, but made difficult.

Additionally, the protective member can provide retaining members which are designed, for example, as notches or recesses for accommodating the widened cross-section of the dose provisioning member. The widened cross-section can, for example, be realized by projections on the dose provisioning member.

In an embodiment, one or more openings on the outer surface of the body of the device, for example an opening formed by the mouthpiece of the device, are covered by a sealing member wherein the sealing member seals the interior of the body against environmental influences. The sealing member is preferably detachable from the openings. The sealing member helps to protect the device from moisture or humidity as well as from dirt which can penetrate through the openings into the interior of the device. Moisture or humidity is in so far dangerous as the dry powder of the medicament can become humid and wet such that powder agglomerations result and can lead to a malfunction of the device or an insufficient air-powder-mixture during inhalation of the medicament. Therefore, it is advantageous to keep the powdered medicament dry within the device. A protection is effected by the sealing member. It is conceivable to provide one single sealing member for all openings or a plurality of sealing members, one for each of the openings.

Another advantage of the sealing member is given by the fact that the device can be stored over a longer period of time before the first use. When a patient wants to use the device, he may detach the sealing member from the opening(s) of the device, e.g. from the mouthpiece of the device, such that an inhalation action through the mouthpiece can be effected.

Moreover, the presence or lack of a sealing member in a device may indicate to a user whether the device has already been used or not. In this context, it is conceivable that the sealing member has a certain warning color, e.g. a red or green color, indicating in the sealed configuration to a user that the device is still untouched.

Additional embodiments, features and advantages are disclosed in the dependent claims, in the figures and in the following description.

In the following, the advantageous design of the device as explained above is described with the aid of several drawings, wherein:
Figure 1 shows a first cross-sectional view of the device in a first configuration,
Figure 2A shows a second cross-sectional view of the device according to Figure 1,
Figure 2B shows the device according to Figure 2A in a second configuration, and
Figure 3 shows the device according to Figure 1 during inhalation by a user.

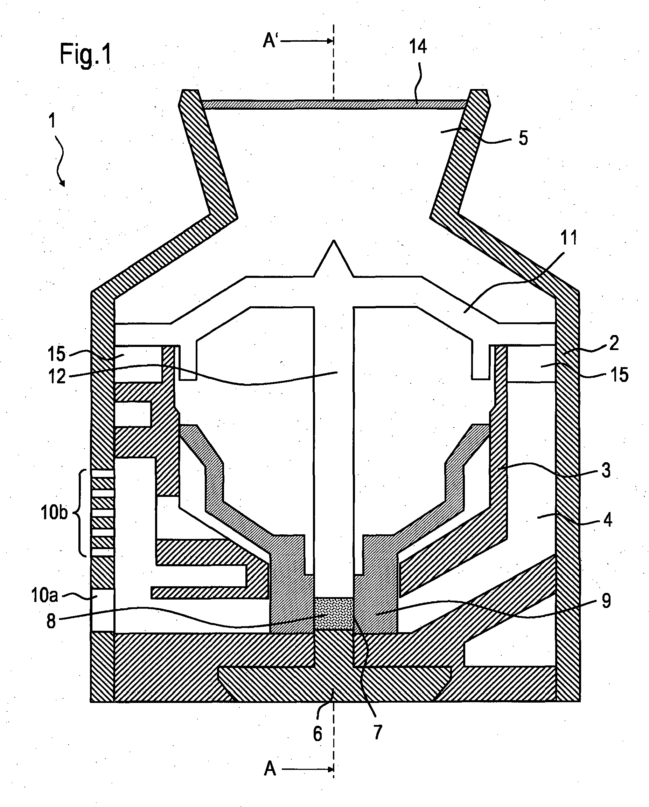

Figure 1 shows a device 1 for administration of a powdered medicament to a patient by inhalation.

The device 1 comprises a body 2 which contains an internal housing 3. The body 2 and the internal housing 3 are designed in the embodiment of Figure 1 as two separate parts. However, it is also conceivable that the body 2 and the internal housing 3 are designed as one single and integrally formed piece.

At the upper end of the body 2 a mouthpiece 5 is integrally formed in the body 2. During use of the device 1 a patient may suck on the mouthpiece 5 in order to inhale a powdered medicament out of the device 1. According to Figure 1, the mouthpiece is sealed by a sealing member 14 which may for example be a detachable cover or foil.

The internal housing 3 is designed such that an inhalation channel 4 is formed by walls of the internal housing 3 and walls of the body 2. In the upper part of the device 1 the inhalation channel 4 is designed as a rotation chamber 15 which is delimited by walls of the housing 3 and the body 2 as well as by walls of a deaggregating member 11 which is also arranged in the interior of the device 1. The deaggregating member 11 together with the rotation chamber 15 is designed for deflecting an airstream within the inhalation channel 4. Moreover, the deaggregating member 11 is designed for separating an airstream in the rotation chamber 15 into several partial airstreams, as explained below. Finally, the inhalation channel 4 ends in the mouthpiece 5 of the device 1.

On the left side of the body 2 several air inlets 10a and 10b are designed such that outside air may enter the device 1. In particular, air may enter the air inlet 10a and may flow through the inhalation channel 4 in an upward direction through the rotation chamber 15 and finally through the mouthpiece 5. The additional air inlets 10b are designed such that outside air may enter the device 1 for actuating a protective member 9 as also explained below.

The device 1 according to Figure 1 also comprises a so-called dose provisioning member 6 which is arranged in a lower section of the device 1. The dose provisioning member 6 is designed as a slider which can be operated by a user. According to the cross-sectional view of Figure 1 the dose provisioning member 6 can be moved in a direction perpendicular to the depicted cross-section of the device 1. This means the dose provisioning member 6 may be moved in a direction perpendicular to the plane of projection of Figure 1. The dose provisioning member 6 is designed with a cavity 7 for accommodation for a dose of a powdered medicament 8. The cavity 7 of the dose provisioning member 6, according to the embodiment of Figure 1, is designed as a recess or hole extending through the sidewalls of the dose provisioning member 6. The total space or volume of the cavity 7 is filled with the dose of powdered medicament 8. But it is also conceivable that only a part of the cavity 7 is filled with the powdered medicament 8.

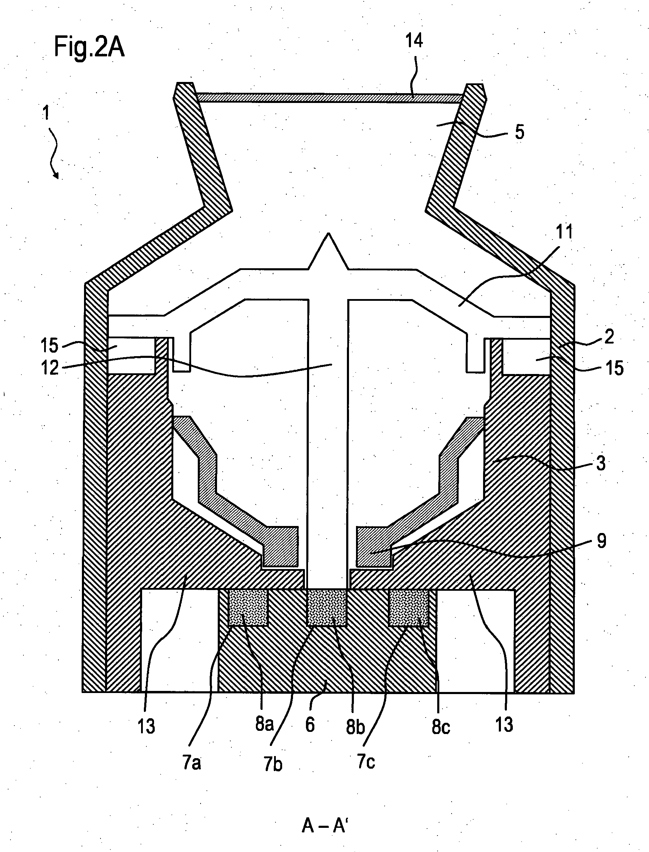

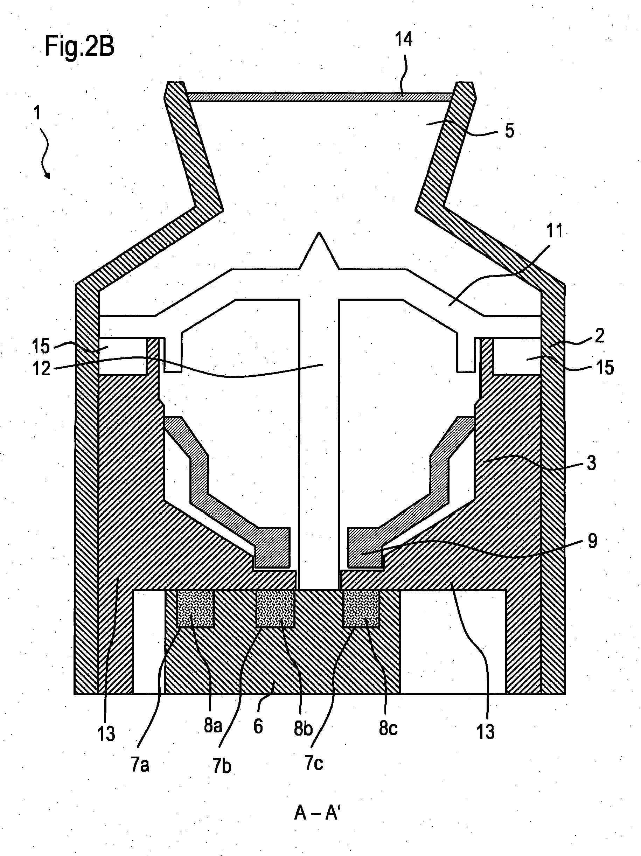

The dose provisioning member 6 can be moved between a first position in which the cavity 7 is out of alignment with the inhalation channel 4 and a second position in which the cavity is in alignment with the inhalation channel 4 as better seen in Figures 2A and 2B. According to the configuration of the device 1 in Figure 1, the dose provisioning member 6 is in its second position, the cavity lying in the inhalation channel 4. Hence, the dose provisioning member 6 is positioned such that the cavity 7, especially the cross-section of the cavity 7, is in alignment with the cross-section of the inhalation channel 4.

As explained above, a protective member 9 is provided in the interior of the device 1, wherein the protective member 9 is movable from a first position into a second position. The protective member 9 is guided by a guide member 12 which is integrally formed on the deaggregating member 11.

According to Figure 1 the protective member 9 is placed in its first position such that a part of the protective member 9 lies in the inhalation channel 4. Consequently, the cross-section of the inhalation channel 4 is covered by the walls of the protective member 9 such that the inhalation channel 4 is blocked by the protective member 9. Moreover, the protective member 9 encloses the cavity 7 with the dose of the powdered medicament prefilled in the cavity 7. Hence, according to Figure 1 the device 1 is in a so-called inhalation-ready position with the dose of the powdered medicament 8 being in alignment with the inhalation channel 4 and being prepared for inhalation. But the inhalation channel 4 is still closed by the protective member 9 such that the dose of the powdered medicament 8 cannot enter the inhalation channel 4.

Figure 2A shows the device 1 according to Figure 1 in a cross-sectional view along the axis A-A'. Figure 2A illustrates the design of the dose provisioning member 6 as a slider in the lower part of the device 1.

The dose provisioning member 6 provides three cavities 7a,7b,7c each of which is filled with a single dose of a powdered medicament 8a,8b,8c. The cavities 7a,7b,7c are designed as recesses extending through the walls of the dose provisioning member 6 as better illustrated regarding Figure 1. According to Figure 2A the cavities 7a,7b,7c are arranged in the dose provisioning member 6 with a predetermined distance between each other. The single doses of the powdered medicament 8a,8b,8c are therefore separated by walls of the dose provisioning member 6. The dose provisioning member 6 is designed for provisioning the three doses of the powdered medicament 8a,8b,8c as three single "shots".

The cavities 7a,7b,7c of the dose provisioning member 6 are prefilled with the powdered medicament 8a,8b,8c without the need of an additional powder reservoir for storing a large amount of the powdered medicament 8. This means that no mechanism is necessary for gathering the powdered medicament 8 in a powder reservoir and for separating a predetermined amount, i.e. a dose of the powdered medicament 8, from the powder reservoir. According to the design of the dose provisioning member 6 the three shots of the powdered medicament 8a,8b,8c are already prepared in the prefilled cavities 7a,7b,7c of the dose provisioning member 6.

According to Figure 2A, the dose provisioning member 6 is positioned in a second position with regard to the dose of the powdered medicament 8b, which is arranged in the middle of the dose provisioning member 6, such that this dose of the powdered medicament 8b is in alignment with the inhalation channel 4 (see Figure 1) which, according to the perspective of Figure 2A, extends in a direction perpendicular to the plane of projection of Figure 2A. In Figure 2A the dose of powdered medicament 8b is situated in the inhalation channel 4 such that the dose of the powdered medicament 8b is ready to be inhaled by a user.

With regard to the other two doses of the powdered medicament 8a and 8c, these doses are enclosed by their respective cavities 7a and 7c and by inner walls 13 of the housing 3. This means that the inner walls 13 of the housing, together with the cavities 7a and 7c of the dose provisioning member 6, form closed chambers which enclose the respective doses of powdered medicament 8a and 8c. In this way the doses of the powdered medicament 8a and 8c are protected against environmental influences as well as from falling out of the cavities 7a and 7c. By moving the dose provisioning member 6, the position of the dose provisioning member 6 relative to the internal housing 3 of the device 1, and especially relative to the inhalation channel 4 as depicted in Figure 1, can be altered.

Regarding Figure 2B, the device 1 according to Figure 2A is depicted with the only difference being that the dose provisioning member 6 has been moved in left direction such that the dose of the powdered medicament 8b has been moved leftwards. In the configuration of Figure 2B the wall of the dose provisioning member 6 lying between the doses 8b and 8c is now in alignment with the inhalation channel 4 as depicted in Figure 1. This means that the cross-section of the inhalation channel 4 is fully covered by the wall of the dose provisioning member 6 according to Figure 2B. Therefore, the dose provisioning member 6 fulfills two functionalities. Besides an accommodation of powdered medicament 8, the dose provisioning member 6 blocks the inhalation channel 4 in the position as depicted in Figure 2B (and also in another position with the wall between the doses 8a and 8b lying in the inhalation channel 4) such that the device 1 is secured against unintentional inhalation. Hence, a safety mechanism is realized in the device 1 by the dose provisioning member 6.

Moreover, the protective member 9 is still in its first position as explained in the context of Figure 1 such that the inhalation channel 4 is additionally covered and blocked by the protective member 9. Furthermore, in the configuration of Figure 2B the protective member 9 lies in the inhalation channel 4 covering the wall of the dose provisioning member 6, which also lies in the inhalation channel 4.

According to the configuration of the device 1 in Figure 2B an unintentional actuation of the device 1 is prevented in two ways. In a first way, the unintentional actuation is prevented by the wall of the dose provisioning member 6 lying in the inhalation channel 4 as explained above. Additionally, an unintentional actuation of the device 1 is also prevented by the protective member 9 lying in the inhalation channel 4 in its first position as depicted in Figure 1.

Moreover, it is conceivable that the dose provisioning member and the protective member interact with each other, such that in the position of the dose provisioning member according to Figure 2B the actuation force required for displacing the protective member from its first position to its second position is greater than in a position of the dose provisioning member with one of the cavities 7a,7b,7c lying in the inhalation channel 4 as exemplary depicted in Figure 2A.

According to one embodiment the interaction between the protective member and the dose provisioning member can be obtained by hook-shaped members interacting with each other. According to another embodiment, the interaction between the protective member and the dose provisioning member can be obtained by a widened cross-section of the dose provisioning member at the part of the dose provisioning member lying in the inhalation channel when the dose provisioning member is in its first position such that this part of the dose provisioning member is in contact with the protective member. In this way the widened cross-section of the dose provisioning member can effect a pressure force against the walls of the protective member within the inhalation channel such that a movement of the protective member against this pressure force is inhibited. "Inhibited" means that a movement is possible, but made difficult.

Additionally, the protective member can provide retaining members which are designed, for example, as notches or recesses for accommodating the widened cross-section of the dose provisioning member. The widened cross-section can, for example, be realized by projections on the dose provisioning member.

When the dose provisioning member 6 is positioned in its position according to Figure 2B all cavities 7a,7b,7c prefilled with doses of the powdered medicament 8a,8b,8c are covered by the inner walls 13 of the internal housing 3. Therefore, the powdered medicament 8a,8b,8c in all cavities 7a,7b,7c of the dose provisioning member 6 is enclosed by the cavities 7a,7b,7c and the inner walls 13 of the internal housing 3.

The dose provisioning member 6 can be operated by a user sliding the dose provisioning member 6 in the left or right direction relative to the internal housing 3 of the device 1. In this way a user may simply and easily prepare the device 1 for inhalation by moving the dose provisioning member 6 from a position as depicted in Figure 2B into a position as depicted in Figure 2A, for example, such that a dose of a powdered medicament 8a,8b,8c can be brought into alignment with the inhalation channel 4 as depicted in Figure 1. It is not illustrated but conceivable that the dose provisioning member 6 is brought into several other positions, thereby bringing the other cavities 7a,7b,7c with the respective doses of the powdered medicament 8a,8b,8c into alignment with the inhalation channel 4.

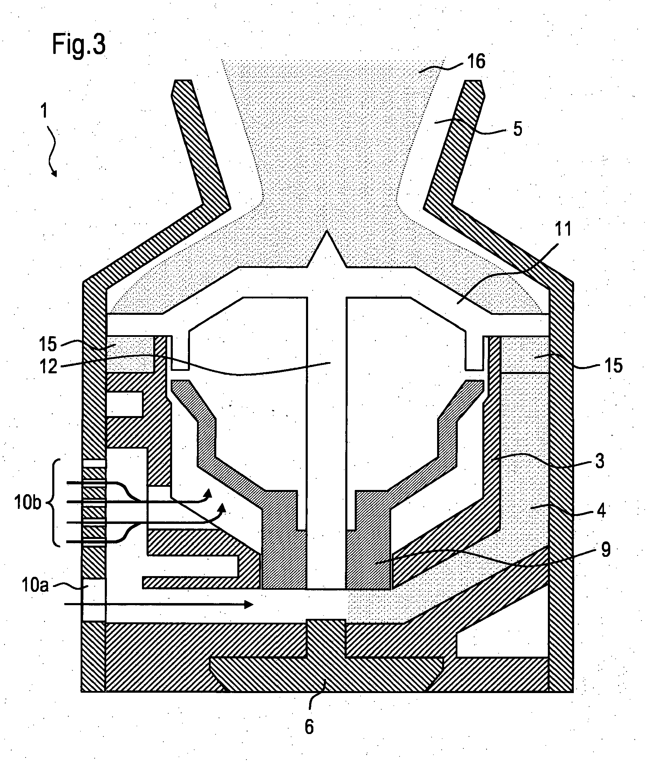

Figure 3 describes an actuation of the device 1 starting from the configuration as depicted in Figure 1.

The dose provisioning member 6 is positioned in the dose provisioning position as depicted in Figure 1, such that a dose of the powdered medicament 8 lies in the inhalation channel 4. When a user wants to actuate the device 1 he has firstly to remove the sealing member 14 in the upper part of the mouthpiece 5 from the device 1. The sealing member 14 can, for example, be a detachable cover or foil protecting the device 1 in its closed position against environmental influences like moisture, humidity or dirt. It is also conceivable to provide a sealing member on the openings 10a and 10b such that the air inlets 10a and 10b are also sealed against environmental influences by a respective sealing member.

Regarding Figure 3, the sealing member 14 has been removed such that the mouthpiece 5 is open in upward direction. A user can now suck on the mouthpiece 5 such that an under-pressure is created by the user. The under-pressure effects a movement of the protective member 9 from its first position, as depicted in Figure 1, to a second position as depicted in Figure 3. Additionally, air flows into the air inlets 10b (as illustrated by arrows in Figure 3) such that the protective member 9 is supported by the streaming air in its upward movement. Hence, the protective member 9 is moved upwards with the aid of a kind of air cushion that is provided by the air flowing into the air inlets 10b.

The protective member 9 is guided by a guide member 12 which is integrally formed on the deaggregating member 11 and by inner walls of the housing 3. A predetermined friction can be provided at a contact area between the protective member 9 and the guide member 12 and/or the inner walls of the housing 3. The friction has the effect that the protective member is not actuated until the pressure difference exceeds a predetermined threshold. Consequently, the protective member 9 is only moved when an intense inhalation action takes place.

The protective member 9 may rest in its upper position after the movement in the upper position. For this purpose, engagement members may be provided at a contact area between the protective member 9 and the guide member 12 or the inner walls of the housing 13 respectively. For resetting the device 1 the protective member 9 can be released from its upper position by e.g. blowing into the device or shaking the device 1.

When the protective member 9 is in its upper position as depicted in Figure 3, the air channel 4 is fully uncovered such that the dose of powdered medicament 8 is released and may enter the inhalation channel 4. Additionally, air entering the air inlet 10a can be guided through the inhalation channel 4, thereby entraining the dose of the powdered medicament 8 out of the cavity 7 of the dose provisioning member 6. In this way an air-powder mixture is guided through the inhalation channel 4 in upward direction into the rotation chamber 15.

The airstream is deflected by the deaggregating member 11 which forms the rotation chamber 15 together with the walls of the internal housing 3 and the body 2. In the interior of the rotation chamber 15 the airstream describes a circular trajectory. It is not illustrated in Figure 3, but conceivable, that tangential slits are provided in the body 2 at the position of the rotation chamber 15 such that additional air may flow into the rotation chamber 15 in order to intensify and emphasise a rotational effect of the airstream in the rotation chamber 15. This may help to create swirl of the airstream such that bigger agglomerates of the powdered medicament can be deaggregated.

Moreover, the deaggregating member 11 is designed to separate the airstream into several airstreams. The separation of the airstream in the rotation chamber 15 by the deaggregating member 11 has also the effect that powder agglomerates are deaggregated such that a uniform and consistent ratio of powder within the airstream is created. Downstream of the deaggregating member 11 in the direction of the mouthpiece 5 the separated airstreams are joined to a uniform spray of the air-powder mixture 16. The air-powder mixture 16 may finally enter the mouth of the patient and may be transported into the lungs of the patient such that the powdered medicament can be absorbed by the alveoli of the patient's lungs.

The device 1 as described and explained above is a simple, cost-effective and easy to use,device which nevertheless provides for a comfortable handling such that single doses of a powdered medicament can be administered to a patient. The device 1 can be assembled with a small number of parts. It is, for example, conceivable to assemble the device 1 with only four, five or six pieces, depending on the molding of the several parts. As the device 1 does not need any powder reservoir for storing the powdered medicament besides the cavities 7 of the dose provisioning member 6 as explained above, the device 1 can be produced in a simple and cost-effective manner.

Moreover, since the dose provisioning member 6fulfills a double functionality (preparation of the powdered medicament 8 as well as a security mechanism against unintentional inhalation) several safety mechanisms can be realized in a cost-effective manner. The device 1 can be easily handled by a user, wherein the dose provisioning member 6 has only to be moved between several positions in order to secure the device or prepare a dose of a powdered medicament for inhalation.

The illustrated embodiments are only exemplary. Hence, it is conceivable to design a device 1 with only one cavity 7 filled with a dose of powdered medicament 8 or with two cavities 7, each of which is filled with a dose of a powdered medicament 8.

The term "medicament", as used herein may mean a pharmaceutical formulation containing at least one pharmaceutically active compound, for example for the treatment of obstructive airway or lung diseases such as asthma or chronic obstructive pulmonary disease (COPD), local respiratory tract oedema, inflammation, viral, bacterial, mycotic or other infection, allergies, diabetes mellitus.

The active pharmaceutical compound is preferably selected from the group consisting of active pharmaceutical compounds suitable for inhalation, preferably antiallergenic, antihistamine, anti-inflammatory, antitussive agents, bronchodilators, anticholinergic drugs, and combinations thereof.

The active pharmaceutical compound may for example be chosen from:
an insulin such as human insulin, e.g. a recombinant human insulin, or a human insulin analogue or derivative, a glucagon-like peptide (GLP-1) or an analogue or derivative thereof, or exendin-3 or exendin-4 or an analogue or derivative of exendin-3 or exendin-4;
an adrenergic agent such as a short acting β2-agonists (e.g. Salbutamol, Albuterol, Levosalbutamol, Fenoterol, Terbutaline, Pirbuterol, Procaterol, Bitolterol, Rimiterol, Carbuterol, Tulobuterol, Reproterol), a long acting β2-agonist (LABA, e.g. Arformoterol, Bambuterol, Clenbuterol, Formoterol, Salmeterol), an ultra LABA (e.g. Indacaterol) or another adrenergic agent (e.g. Epinephrine, Hexoprenaline, Isoprenaline (Isoproterenol), Orciprenaline (Metaproterenol));
a glucocorticoid (e.g. Beclometasone, Budesonide, Ciclesonide, Fluticasone, Mometasone, Flunisolide, Betamethasone, Triamcinolone);
an anticholinergic agent or muscarinic antagonist (e.g. Ipratropium bromide, Oxitropium bromide, Tiotropium bromide);
a mast cell stabilizer (e.g. Cromoglicate, Nedocromil);
a xanthine derivative (e.g. Doxofylline, Enprofylline, Theobromine, Theophylline, Aminophylline, Choline theophyllinate);
an eicosanoid inhibitor, such as a leukotriene antagonist (e.g. Montelukast, Pranlukast, Zafirlukast), a lipoxygenase inhibitor (e.g. Zileuton) or a thromboxane receptor antagonist (e.g. Ramatroban, Seratrodast);
a phosphodiesterase type-4 inhibitor (e.g. Roflumilast);
an antihistamine (e.g. Loratadine, Desloratadine, Cetirizen, Levocetirizine, Fexofenadine);
an allergen immunotherapy (e.g. Omalizumab);
a mucolytic (e.g. Carbocisteine, Erdosteine, Mecysteine);
an antibiotic or antimycotic;
or a combination of any two, three or more of the above-mentioned compound classes or compounds (e.g. Budesonide/Formoterol, Fluticasone/Salmeterol, Ipratropium bromide/Salbutamol, Mometasone/Formoterol);
or a pharmaceutically acceptable salt or solvate or esters of any of the above named compounds.

Pharmaceutically acceptable salts are for example acid addition salts and basic salts. Acid addition salts are e.g. a chloride, bromide, iodide, nitrate, carbonate, sulfate, methylsulfate, phosphate, acetate, benzoate, benzenesulfonate, fumarate, malonate, tartrate, succinate, citrate, lactate, gluconate, glutamate, edetate, mesylate, pamoate, pantothenate or a hydroxy-naphthoate salt. Basic salts are for example salts having a cation selected from alkali or alkaline, e.g. Na+, or K+, or Ca2+, or an ammonium ion N+(R1)(R2)(R3)(R4), wherein R1 to R4 independently of each other mean: hydrogen, an optionally substituted C1-C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology. Pharmaceutically acceptable ester may for example be acetates, propionates, phosphates, succinates or etabonates.

Pharmaceutically acceptable solvates are for example hydrates.

### Reference numerals

- 1: device
- 2: body
- 3: internal housing
- 4: inhalation channel
- 5: mouthpiece
- 6: dose provisioning member
- 7,7a,7b,7c: cavity
- 8,8a,8b,8c: dose of powdered medicament
- 9: protective member
- 10a,10b: air inlets
- 11: deaggregating member
- 12: guide member
- 13: inner walls of housing
- 14: sealing member
- 15: rotation chamber

## Claims

1. A device (1) for administering a powdered medicament to a patient by inhalation, the device (1) comprising:
- a body (2),
- an internal housing (3) in the body (2),
- an inhalation channel (4),
- a mouthpiece (5), the inhalation channel (4) being provided to communicate with the mouthpiece (5), and
- a dose provisioning member (6), the dose provisioning member (6) comprising at least one cavity (7,7a,7b,7c) for accommodation of a powdered medicament, the dose provisioning member (6) further being enabled to adopt a first position in which the cavity (7,7a,7b,7c) is out of alignment with the inhalation channel (4) and a second position in which the cavity (7,7a,7b,7c) is in alignment with the inhalation channel (4), wherein each cavity (7,7a,7b,7c) is prefilled with a single dose (8,8a,8b,8c) of a powdered medicament, and wherein the powdered medicament is solely stored in each cavity (7,7a,7b,7c) and the device (1) is free of a powder reservoir for storing a plurality of doses,
**characterized in that**
a protective member (9) is provided within the housing (3), the protective member (9) being enabled to adopt
- a first position in which a part of the protective member (9) lies in the inhalation channel (4) such that the cross-section of the inhalation channel (4) is covered by a wall of the protective member (9) and
- a second position in which the protective member (9) does not lie in the inhalation channel (4) such that the cross-section of the inhalation channel (4) is not covered by a wall of the protective member (9).

2. The device (1) according to claim 1, **characterized in that**,
in the first position of the dose provisioning member (6) a part of the dose provisioning member (6) lies in the inhalation channel (4) such that the inhalation channel (4) is at least partly covered by a wall of the dose provisioning member (6).

3. The device (1) according to claim 1 or 2, **characterized in that** in the first position the cavity (7,7a,7b,7c) is covered by inner walls (13) of the housing (3) such that the powdered medicament is enclosed by the cavity (7,7a,7b,7c) and the inner walls (13) of the housing (3).

4. The device (1) according to one of claims 1 to 3, **characterized in that** the cavity (7,7a,7b,7c) extends through the dose provisioning member (6), wherein in the second position of the dose provisioning member (6) the cavity (7,7a,7b,7c) lies in the inhalation channel (4) such that a cross-section of the cavity (7,7a,7b,7c) taken along the direction of movement of the dose provisioning member (6) between the first and the second position is in alignment with the cross-section of the inhalation channel (4).

5. The device (1) according to one of claims 1 to 4, **characterized in that** in the first position of the protective member (9), the protective member (9) encloses
- a part of the dose provisioning member (6) when the dose provisioning member (6) is in its first position, and
- the cavity (7,7a,7b,7c) when the dose provisioning member (6) is in its second position.

6. The device (1) according to one of claims 1 to 5, **characterized in that** the direction of movement of the protective member (9) between its first and second position is oblique, notably perpendicular, to the direction of movement of the dose provisioning member (6) between its first and second position.

7. The device (1) according to one of claims 1 to 6, **characterized in that** the protective member (9) is designed to be moved from the first position into the second position due to a predetermined pressure difference applicable by a user via the mouthpiece.

8. The device (1) according to claim 7, **characterized in that** the protective member (9) is guided by a guide member (12), wherein a predetermined friction is provided at a contact area between the protective member (1) and the guide member (12) such that a movement of the protective member (9) from the first position into the second -position is only allowed if the pressure difference exceeds a predetermined threshold.

9. The device (1) according to one of claims 1 to 8, **characterized in that** the dose provisioning member (6) and the protective member (9) interact with each other, such that in the first position of the dose provisioning member (6) the actuation force required for displacing the protective member (9) from its first position to its second position is greater than in the second position of the dose provisioning member.

10. The device (1) according to one of claims 1 to 9, **characterized in that** the dose provisioning member (6) is designed as a slider which is operable by a user.

11. The device (1) according to one of claims 1 to 10, **characterized in that** the inhalation channel (4) extends from an air inlet (10a, 10b) in the body (2) through the housing (3) to the mouthpiece (5), wherein an airstream can be guided in the inhalation channel (4) from the air inlet (10a, 10b) to the mouthpiece (5) during inhalation by a patient.

12. The device (1) according to one of claims 1 to 11, **characterized in that** a deaggregating member (11) is provided in the inhalation channel (4) between the dose provisioning member (6) and the mouthpiece (5), the deaggregating member (11) being designed to deflect the airstream.

13. The device (1) according to claim 12, **characterized in that** the deaggregating member (11) is designed to separate the airstream into several partial airstreams.

14. The device (1) according to one of claims 1 to 13, **characterized in that** one or more openings on the outer surface of the body (2) are covered by a sealing member (14), sealing the interior of the body (2) against environmental influences, wherein the sealing member (14) is detachable from the openings.

## Patentansprüche

1. Vorrichtung (1) zur Verabreichung eines pulverförmigen Medikaments an einen Patienten durch Inhalation, wobei die Vorrichtung (1) Folgendes umfasst:
- einen Korpus (2),
- ein internes Gehäuse (3) im Korpus (2),
- einen Inhalationskanal (4),
- ein Mundstück (5), wobei der Inhalationskanal (4) zur Kommunikation mit dem Mundstück (5) eingerichtet ist, und
- ein Dosisbereitstellungsglied (6), wobei das Dosisbereitstellungsglied (6) mindestens einen Hohlraum (7, 7a, 7b, 7c) zur Aufnahme eines pulverförmigen Medikaments umfasst, wobei das Dosisbereitstellungsglied (6) ferner in der Lage ist, eine erste Position, in der der Hohlraum (7, 7a, 7b, 7c) nicht fluchtend zum Inhalationskanal (4) ausgerichtet ist, und eine zweite Position, in der der Hohlraum (7, 7a, 7b, 7c) fluchtend zum Inhalationskanal (4) ausgerichtet ist, einzunehmen, wobei jeder Hohlraum (7, 7a, 7b, 7c) mit einer einzelnen Dosis (8, 8a, 8b, 8c) eines pulverförmigen Medikaments vorbefüllt ist und wobei das pulverförmige Medikament ausschließlich in jedem Hohlraum (7, 7a, 7b, 7c) gelagert ist und die Vorrichtung (1) kein Pulverreservoir zur Lagerung einer Vielzahl von Dosen aufweist,
**dadurch gekennzeichnet, dass**
ein Schutzglied (9) im Gehäuse (3) vorgesehen ist, wobei das Schutzglied (9) in der Lage ist,
- eine erste Position, in der ein Teil des Schutzglieds (9) so im Inhalationskanal (4) liegt, dass der Querschnitt des Inhalationskanals (4) durch eine Wand des Schutzglieds (9) abgedeckt ist, und
- eine zweite Position, in der das Schutzglied (9) nicht im Inhalationskanal (4) liegt, so dass der Querschnitt des Inhalationskanals (4) nicht durch eine Wand des Schutzglieds (9) abgedeckt ist, einzunehmen.

2. Vorrichtung (1) nach Anspruch 1, **dadurch gekennzeichnet, dass** in der ersten Position des Dosisbereitstellungsglieds (6) ein Teil des Dosisbereitstellungsglieds (6) so im Inhalationskanal (4) liegt, dass der Inhalationskanal (4) mindestens teilweise durch eine Wand des Dosisbereitstellungsglieds (6) abgedeckt ist.

3. Vorrichtung (1) nach Anspruch 1 oder 2, **dadurch gekennzeichnet, dass** der Hohlraum (7, 7a, 7b, 7c) in der ersten Position durch innere Wände (13) des Gehäuses (3) abgedeckt ist, so dass das pulverförmige Medikament durch den Hohlraum (7, 7a, 7b, 7c) und die inneren Wände (13) des Gehäuses (3) umschlossen ist.

4. Vorrichtung (1) nach einem der Ansprüche 1 bis 3, **dadurch gekennzeichnet, dass** sich der Hohlraum (7, 7a, 7b, 7c) durch das Dosisbereitstellungsglied (6) erstreckt, wobei der Hohlraum (7, 7a, 7b, 7c) in der zweiten Position des Dosisbereitstellungsglieds (6) so im Inhalationskanal (4) liegt, dass ein entlang der Bewegungsrichtung des Dosisbereitstellungsglieds (6) zwischen der ersten und der zweiten Position orientierter Querschnitt des Hohlraums (7, 7a, 7b, 7c) fluchtend zum Querschnitt des Inhalationskanals (4) ausgerichtet ist.

5. Vorrichtung (1) nach einem der Ansprüche 1 bis 4, **dadurch gekennzeichnet, dass** in der ersten Position des Schutzglieds (9) das Schutzglied (9)
- einen Teil des Dosisbereitstellungsglieds (6) umschließt, wenn das Dosisbereitstellungsglied (6) in seiner ersten Position ist, und
- den Hohlraum (7, 7a, 7b, 7c) umschließt, wenn das Dosisbereitstellungsglied (6) in seiner zweiten Position ist.

6. Vorrichtung (1) nach einem der Ansprüche 1 bis 5, **dadurch gekennzeichnet, dass** die Bewegungsrichtung des Schutzglieds (9) zwischen seiner ersten und zweiten Position schräg, insbesondere senkrecht, zu der Bewegungsrichtung des Dosisbereitstellungsglieds (6) zwischen seiner ersten und zweiten Position verläuft.

7. Vorrichtung (1) nach einem der Ansprüche 1 bis 6, **dadurch gekennzeichnet, dass** das Schutzglied (9) dazu ausgelegt ist, aufgrund eines vorbestimmten Druckunterschieds, der durch einen Benutzer über das Mundstück aufbringbar ist, aus der ersten Position in die zweite Position bewegt zu werden.

8. Vorrichtung (1) nach Anspruch 7, **dadurch gekennzeichnet, dass** das Schutzglied (9) durch ein Führungsglied (12) geführt ist, wobei eine vorbestimmte Reibung an einem Kontaktbereich zwischen dem Schutzglied (1) und dem Führungsglied (12) bereitgestellt ist, so dass eine Bewegung des Schutzglieds (9) aus der ersten Position in die zweite Position nur gestattet ist, wenn der Druckunterschied eine vorbestimmte Schwelle überschreitet.

9. Vorrichtung (1) nach einem der Ansprüche 1 bis 8, **dadurch gekennzeichnet, dass** das Dosisbereitstellungsglied (6) und das Schutzglied (9) zusammenwirken, so dass die zum Verschieben des Schutzglieds (9) aus seiner ersten Position in seine zweite Position erforderliche Betätigungskraft in der ersten Position des Dosisbereitstellungsglieds (6) größer als in der zweiten Position des Dosisbereitstellungsglieds ist.

10. Vorrichtung (1) nach einem der Ansprüche 1 bis 9, **dadurch gekennzeichnet, dass** das Dosisbereitstellungsglied (6) als ein von einem Benutzer betätigbarer Schieber ausgelegt ist.

11. Vorrichtung (1) nach einem der Ansprüche 1 bis 10, **dadurch gekennzeichnet, dass** sich der Inhalationskanal (4) von einem Lufteinlass (10a, 10b) im Korpus (2) durch das Gehäuse (3) zum Mundstück (5) erstreckt, wobei während der Inhalation durch einen Patienten ein Luftstrom im Inhalationskanal (4) vom Lufteinlass (10a, 10b) zum Mundstück (5) geführt werden kann.

12. Vorrichtung (1) nach einem der Ansprüche 1 bis 11, **dadurch gekennzeichnet, dass** ein Deaggregationsglied (11) im Inhalationskanal (4) zwischen dem Dosisbereitstellungsglied (6) und dem Mundstück (5) vorgesehen ist, wobei das Deaggregationsglied (11) zum Umlenken des Luftstroms ausgelegt ist.

13. Vorrichtung (1) nach Anspruch 12, **dadurch gekennzeichnet, dass** das Deaggregationsglied (11) dazu ausgelegt ist, den Luftstrom in mehrere Teilluftströme zu zerlegen.

14. Vorrichtung (1) nach einem der Ansprüche 1 bis 13, **dadurch gekennzeichnet, dass** eine oder mehrere Öffnungen an der Außenfläche des Körpers (2) durch ein Dichtglied (14) abgedeckt sind, das das Innere des Körpers (2) gegen Umwelteinflüsse abdichtet, wobei das Dichtglied (14) von den Öffnungen ablösbar ist.

## Revendications

1. Dispositif (1) destiné à administrer un médicament en poudre à un patient par inhalation, le dispositif (1) comportant :
- un corps (2),
- un logement (3) interne dans le corps (2),
- un canal (4) d'inhalation,
- un embout buccal (5), le canal (4) d'inhalation étant fourni pour communiquer avec l'embout buccal (5), et
- un élément (6) de fourniture de dose, l'élément (6) de fourniture de dose comportant au moins une cavité (7, 7a, 7b, 7c) destinée à loger un médicament en poudre, l'élément (6) de fourniture de dose étant en outre capable d'adopter une première position dans laquelle la cavité (7, 7a, 7b, 7c) n'est pas alignée avec le canal (4) d'inhalation et une seconde position dans laquelle la cavité (7, 7a, 7b, 7c) est alignée avec le canal (4) d'inhalation,
où chaque cavité (7, 7a, 7b, 7c) est préremplie d'une seule dose (8, 8a, 8b, 8c) d'un médicament en poudre, et où le médicament en poudre est uniquement stocké dans chaque cavité (7, 7a, 7b, 7c) et le dispositif (1) est exempt de réservoir de poudre pour le stockage d'une pluralité de doses,
**caractérisé en ce que**
un élément (9) de protection est fourni à l'intérieur du logement (3), l'élément (9) de protection étant capable d'adopter
- une première position dans laquelle une partie de l'élément (9) de protection repose dans le canal (4) d'inhalation de sorte que la section transversale du canal (4) d'inhalation est recouverte par une paroi de l'élément (9) de protection et
- une seconde position dans laquelle l'élément (9) de protection ne repose pas dans le canal (4) d'inhalation de sorte que la section transversale du canal (4) d'inhalation n'est pas recouverte par une paroi de l'élément (9) de protection.

2. Dispositif (1) selon la revendication 1, **caractérisé en ce que**,
dans la première position de l'élément (6) de fourniture de dose, une partie de l'élément (6) de fourniture de dose repose dans le canal (4) d'inhalation de sorte que le canal (4) d'inhalation est au moins partiellement recouvert par une paroi de l'élément (6) de fourniture de dose.

3. Dispositif (1) selon la revendication 1 ou 2, **caractérisé en ce que** dans la première position la cavité (7, 7a, 7b, 7c), est recouverte par les parois (13) internes du logement (3) de sorte que le médicament en poudre est enfermé par la cavité (7, 7a, 7b, 7c) et les parois (13) internes du logement (3).

4. Dispositif (1) selon l'une quelconque des revendications 1 à 3, **caractérisé en ce que** la cavité (7, 7a, 7b, 7c) s'étend dans l'élément (6) de fourniture de dose, où dans la seconde position de l'élément (6) de fourniture de dose la cavité (7, 7a, 7b, 7c) repose dans le canal (4) d'inhalation de sorte qu'une section transversale de la cavité (7, 7a, 7b, 7c) prise le long de la direction du mouvement de l'élément (6) de fourniture de dose entre la première et la seconde position est alignée avec la section transversale du canal (4) d'inhalation.

5. Dispositif (1) selon l'une quelconque des revendications 1 à 4, **caractérisé en ce que** dans la première position de l'élément (9) de protection, l'élément (9) de protection enferme
- une partie de l'élément (6) d'apport de dose lorsque l'élément (6) d'apport de dose est dans sa première position, et
- la cavité (7, 7a, 7b, 7c) lorsque l'élément (6) d'apport de dose est dans sa seconde position.

6. Dispositif (1) selon l'une quelconque des revendications 1 à 5, **caractérisé en ce que** la direction du mouvement de l'élément (9) de protection entre sa première et sa seconde position est oblique, plus particulièrement perpendiculaire, à la direction du mouvement de l'élément (6) d'apport de dose entre sa première et sa seconde position.

7. Dispositif (1) selon l'une quelconque des revendications 1 à 6, **caractérisé en ce que** l'élément (9) de protection est conçu pour être déplacé depuis la première position dans la seconde position à cause d'une différence de pression prédéterminée pouvant être appliquée par un utilisateur par l'intermédiaire de l'embout buccal.

8. Dispositif (1) selon la revendication 7, **caractérisé en ce que** l'élément (9) de protection est guidé par un élément (12) de guidage, où un frottement prédéterminé est fourni au niveau d'une zone de contact entre l'élément (1) de protection et l'élément (12) de guidage de sorte qu'un mouvement de l'élément (9) de protection de la première position dans la seconde position n'est seulement autorisé que si la différence de pression dépasse un seuil prédéterminé.

9. Dispositif (1) selon l'une quelconque des revendications 1 à 8, **caractérisé en ce que** l'élément (6) de fourniture de dose et l'élément (9) de protection agissent l'un sur l'autre, de sorte que dans la première position de l'élément (6) de fourniture de dose la force d'actionnement requise pour déplacer l'élément (9) de protection de sa première position à sa seconde position est supérieure à celle dans la seconde position de l'élément de fourniture de dose.

10. Dispositif (1) selon l'une quelconque des revendications 1 à 9, **caractérisé en ce que** l'élément (6) de fourniture de dose est conçu comme coulisseau pouvant être utilisé par un utilisateur.

11. Dispositif (1) selon l'une quelconque des revendications 1 à 10, **caractérisé en ce que** le canal (4) d'inhalation s'étend d'une entrée (10a, 10b) dans le corps (2) à travers le logement (3) jusqu'à l'embout buccal (5), où un courant d'air peut être guidé dans le canal (4) d'inhalation depuis l'entrée (10a, 10b) d'air jusqu'à l'embout buccal (5) durant l'inhalation par un patient.

12. Dispositif (1) selon l'une quelconque des revendications 1 à 11, **caractérisé en ce qu'**un élément (11) de désagrégation est fourni dans le canal (4) d'inhalation entre l'élément (6) de fourniture de dose et l'embout buccal (5), l'élément (11) de désagrégation étant conçu pour faire dévier le courant d'air.

13. Dispositif (1) selon la revendication 12, **caractérisé en ce que** l'élément (11) de désagrégation est conçu pour séparer le courant d'air en plusieurs courants d'air partiaux.

14. Dispositif (1) selon l'une quelconque des revendications 1 à 13, **caractérisé en ce qu'**une ou plusieurs ouvertures sur la surface externe du corps (2) sont recouvertes par un élément (14) d'étanchéité, assurant l'étanchéité de l'intérieur du corps (2) contre les influences environnementales, où l'élément (14) d'étanchéité peut être détaché des ouvertures.
